# EUROPEAN PATENT SPECIFICATION

(11) **EP 4 572 844 B1**
(45) Date of publication and mention of the grant of the patent: **03.12.2025**
(21) Application number: 23757599.8
(22) Date of filing: 16.08.2023
(51) Int. Cl.: A61N 1/39, F16M 11/04

(54) **DEVICE FOR FIXING DEFIBRILLATOR**
VORRICHTUNG ZUR FIXIERUNG EINES DEFIBRILLATORS
DISPOSITIF DE FIXATION DE DEFIBRILLATEUR

(30) Priority: 18.08.2022 CN 202210991692; 18.08.2022 CN 202222183049 U
(43) Date of publication of application: 25.06.2025
(73) Proprietor: Koninklijke Philips N.V., 5656 AG Eindhoven (NL)
(72) Inventor: YU, Shangyouqiao, 5656 AG Eindhoven (NL); WU, Jikun, 5656 AG Eindhoven (NL); YANG, Chao, 5656AG Eindhoven (NL)
(74) Representative: Philips Intellectual Property & Standards
(86) International application number: PCT/EP2023/072493
(87) International publication number: WO 2024/038066

(56) References cited:
- EP-A1- 1 365 187
- CN-A- 114 754 236
- DE-A1- 102017 110 001
- DE-U1- 202020 105 649
- US-A- 3 693 921
- US-A- 4 466 595
- US-A1- 2006 142 808
- US-A1- 2015 267 858
- US-A1- 2021 025 544

## Description

### TECHNICAL FIELD

The present invention relates to a defibrillator, in particular to a device for fixing a defibrillator.

### BACKGROUND

In case of emergency, a defibrillator needs to be placed on an ambulance and then transported to the site and taken out quickly. In the process of transporting a patient to a hospital, it is necessary to quickly and conveniently fix the defibrillator on the ambulance and keep the defibrillator running, so as to monitor, defibrillate, and pace the patient. In this process, how to quickly and conveniently fix the defibrillator firmly on the ambulance is critical to successfully saving the life of the patient. An existing device for fixing a defibrillator is usually complex in structure and complicated in operation, and it is therefore difficult to quickly and conveniently fix the defibrillator firmly on an ambulance.

Therefore, the device for fixing a defibrillator in the prior art, as for example shown in EP1365187A1, needs to be improved.

### SUMMARY

An objective of the present invention is to provide a device for fixing a defibrillator. The device for fixing a defibrillator can overcome at least one of the defects in the prior art.

According to one aspect of the present invention, provided is a device for fixing a defibrillator, comprising:
a base assembly; and
an adapter plate detachably mounted on the base assembly, the adapter plate being fixedly mounted on the defibrillator,
wherein the base assembly comprises:
   a base comprising a base plate;
   a movable unit, the adapter plate being detachably mounted on the movable unit;
   an actuating mechanism mounted on the base and configured to drive the movable unit toward the outside of the base;
   a locking mechanism for locking the movable unit moved to the inside of the base in place; and
a stop mechanism for preventing the adapter plate mounted on the movable unit from moving.

According to claim 1, the movable unit comprises a support plate for mounting the adapter plate; the actuating mechanism comprises a guide rod fixedly supported on the base plate, a slide block slidably arranged on the guide rod, and a first spring sheathed on the guide rod and exerting a spring force on the slide block so as to push the slide block along the guide rod; and
the support plate is fixed on the slide block so as to move together with the slide block.

Preferably, the locking mechanism comprises a locking block arranged on the base plate, a locking rod mounted on the movable unit and capable of engaging with the locking block, and an operating component for operating the locking rod to disengage the locking rod from the engagement with the locking block.

Preferably, the locking mechanism comprises:
two locking blocks arranged on the base plate;
two L-shaped locking rods mounted on the movable unit, each of the L-shaped locking rods being provided with a hook at a first end close to the corresponding locking block, and each of the L-shaped locking rods being pivotally mounted on the movable unit at a corner;
a pressing component mounted on the movable unit and capable of pressing a second end of each of the L-shaped locking rods; and
second springs acting on each of the L-shaped locking rods respectively, the second spring enabling each of the L-shaped locking rods to pivot so that the hook is hooked on the corresponding locking block.

Preferably, the support plate is provided with a handle, and each of the L-shaped locking rods and the pressing component are mounted on the handle.

Preferably, the pressing component is arranged on the inside of the handle.

Preferably, the base further comprises side plates fixedly arranged at both sides of the base plate, and the stop mechanism comprises:
convex pillars arranged on the support plate, the convex pillars being inserted into positioning holes formed on the adapter plate to limit the adapter plate from horizontally moving relative to the support plate; and
stop rods arranged on each of the side plates respectively and extending toward the inside of the base, the stop rod having a certain distance from the support plate in a vertical direction to allow the movable unit and the adapter plate mounted on the movable unit to horizontally move, and limit the adapter plate from vertically moving relative to the support plate when the movable unit and the adapter plate move to the inside of the base.

Preferably, a top end of the convex pillar may be designed to be hemispherical.

Preferably, the adapter plate is designed to be I-shaped, each arm of the adapter plate is provided with a stepped recess, and when the movable unit and the adapter plate are moved to the inside of the base, the stop rod is respectively received in the corresponding stepped recess.

Preferably, the support plate is provided with a guide groove corresponding to the outer contour of the adapter plate.

Preferably, a wire guard is arranged at a rear end of the base, and a wire hole is formed on the wire guard.

Preferably, the base is provided with a dustproof wall, and the dustproof wall, the base, and the support plate together define a closed space for accommodating the actuating mechanism.

Preferably, the device for fixing a defibrillator further comprises an independent lock for preventing the movable unit from being accidentally ejected, the movable unit further comprises a front-end plate arranged at a front-end of the support plate, and the independent lock comprises a bolt arranged on the front-end plate and a stop block arranged on the base plate and configured to block the bolt.

The device for fixing a defibrillator, provided in the present invention, is simple in structure and easy to operate, and can quickly and conveniently fix the defibrillator firmly on an ambulance.

### DESCRIPTION OF THE DRAWINGS

FIG. 1 schematically shows a device for fixing a defibrillator according to the present invention by a three-dimensional exploded view, and FIG. 1 does not show a handle and components of a locking mechanism mounted on the handle.
FIG. 2 schematically shows a base assembly of a device for fixing a defibrillator according to the present invention by a three-dimensional view, wherein a movable unit of the base assembly is removed to show internal structures.
FIG. 3 is a three-dimensional view similar to FIG. 2, but a wire guard is additionally arranged.
FIG. 4 schematically shows a movable unit of a device for fixing a defibrillator according to the present invention by a three-dimensional view.
FIG. 5 schematically shows the movable unit in FIG. 4 by a bottom three-dimensional view.
FIG. 6 schematically shows a base assembly of a device for fixing a defibrillator according to the present invention by a three-dimensional view, wherein the base assembly is partially split to show internal structures.
FIG. 7 schematically shows an independent lock of a device for fixing a defibrillator according to the present invention by a partial three-dimensional view.
FIG. 8 schematically shows an adapter plate of a device for fixing a defibrillator according to the present invention, mounted on a bottom of the defibrillator, by a three-dimensional view.
FIG. 9 schematically shows a device for fixing a defibrillator according to the present invention by a three-dimensional view, wherein a movable unit is ejected from a base and an adapter plate is not placed on the movable unit.
FIG. 10 is a top view of the device for fixing a defibrillator shown in FIG. 9.
FIG. 11 schematically shows a device for fixing a defibrillator according to the present invention by a three-dimensional view, wherein a movable unit is inserted into a base and an adapter plate is fixedly held on the movable unit.

### DETAILED DESCRIPTION

The specific embodiments of the present invention will be described in detail below with reference to the drawings. It should be understood that the drawings are only used for illustrating the present invention, but do not constitute a limitation of the present invention.

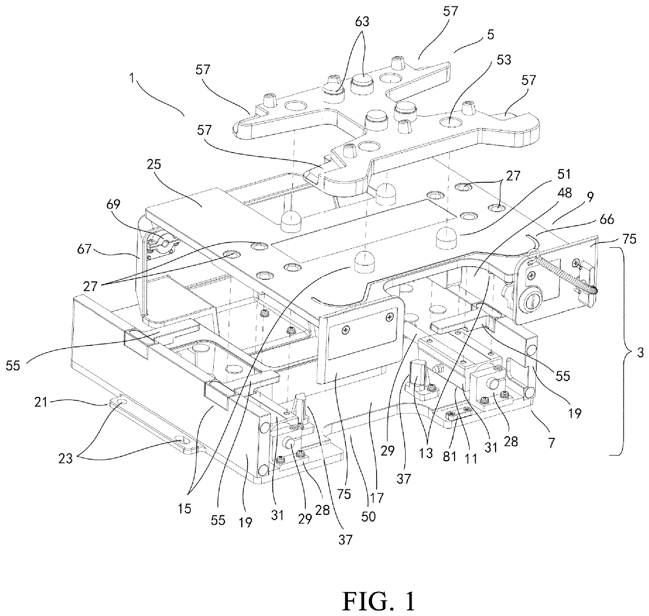

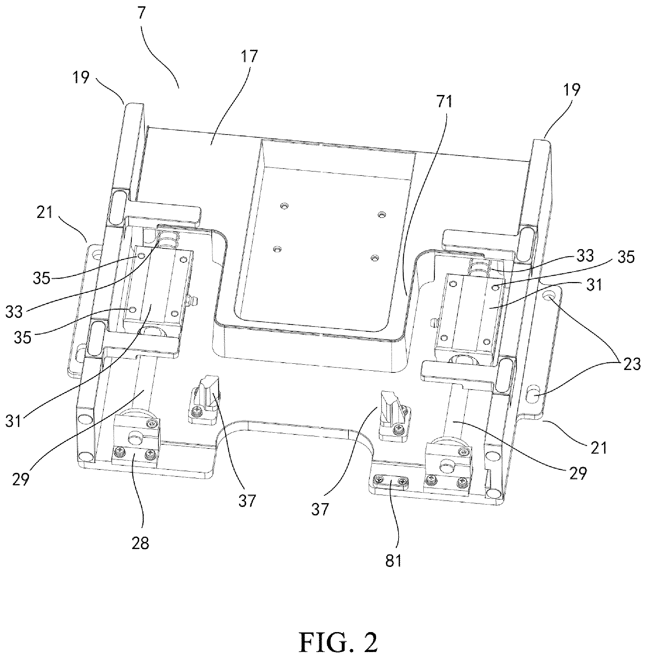

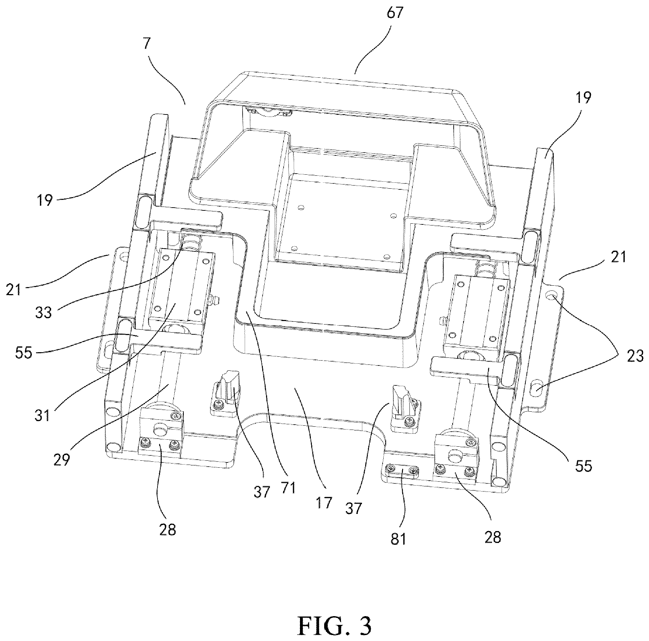

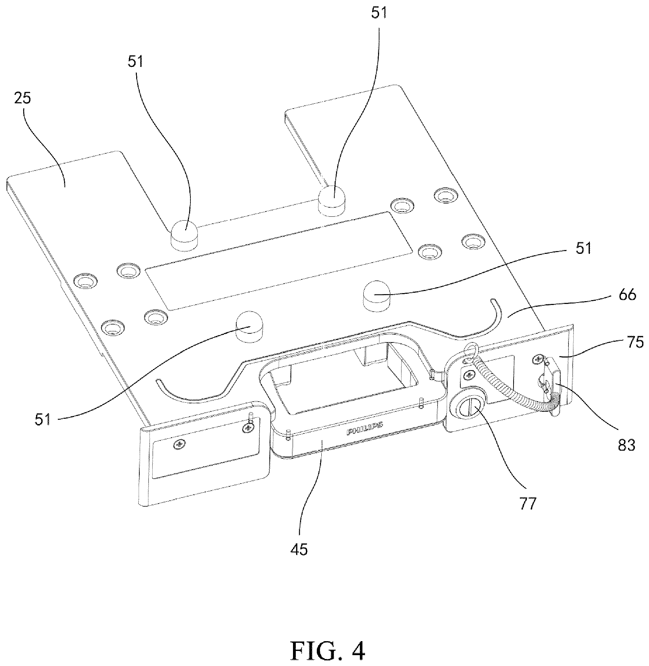

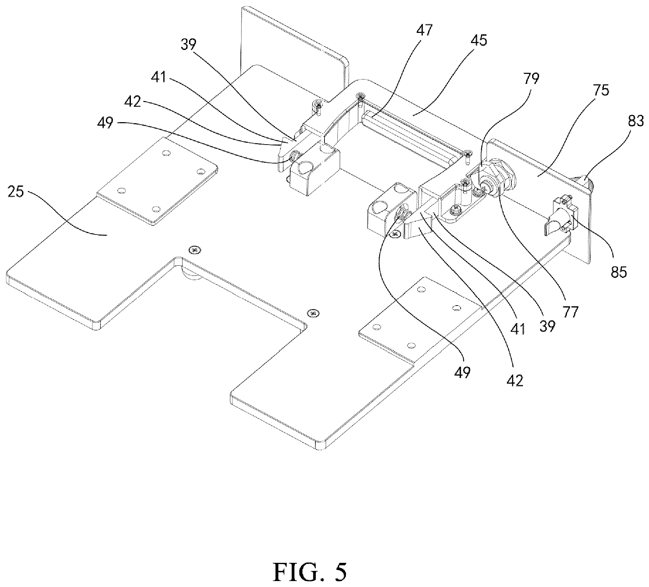

FIG. 1 schematically shows a device for fixing a defibrillator according to the present invention by a three-dimensional exploded view, and FIG. 1 does not show a handle and components of a locking mechanism mounted on the handle. FIG. 2 schematically shows a base assembly of a device for fixing a defibrillator according to the present invention by a three-dimensional view, wherein a movable unit of the base assembly is removed to show internal structures. FIG. 3 is a three-dimensional view similar to FIG. 2, but a wire guard is additionally arranged. FIG. 4 schematically shows a movable unit of a device for fixing a defibrillator according to the present invention by a three-dimensional view. FIG. 5 schematically shows the movable unit in FIG. 4 by a bottom three-dimensional view. As shown in FIG. 1 to FIG. 5, a device 1 for fixing a defibrillator according to the present invention generally includes a base assembly 3 and an adapter plate 5 detachably held on the base assembly 3. The base assembly 3 includes a base 7, a movable unit 9, an actuating mechanism 11 for driving the movable unit 9 toward the outside of the base 7, a locking mechanism 13 for locking the movable unit 9 moved to the inside of the base 7 in place, and a stop mechanism 15 for preventing the adapter plate 5 mounted on the movable unit 9 from moving.

As shown in FIG. 1 to FIG. 3, the base 7 includes a base plate 17 and side plates 19 fixedly arranged at both sides of the base plate 17. The base plate 17 can be fixedly mounted on an ambulance. For example, both sides of the base plate 17 can be provided with side wings 21 protruding relative to the side plates 19, each side wing 21 is provided with a through hole 23, and the base plate 17 can be fixedly mounted on the ambulance by screws (not shown) passing through the through holes 23.

As shown in FIG. 1, FIG. 4 and FIG. 5, the movable unit 9 includes a support plate 25 which can be driven by the actuating mechanism 11 and is substantially flat, and a plurality of mounting holes 27 are respectively formed on both sides of the support plate 25. As shown in FIG. 1, FIG. 2 and FIG. 3, the actuating mechanism 11 for driving the movable unit 9 toward the outside of the base 7 includes a guide rod 29 with both ends fixedly supported on the base plate 17 by a support seat 28, a slide block 31 through which the guide rod 29 passes so as to enable the slide block to slide along the guide rod 29, and a first spring 33 sheathed on the guide rod 29 and exerting a spring force on the slide block 31 so as to push the slide block 31 to the outside of the base 7 along the guide rod 29. The actuating mechanism 11 including the guide rod 29, the slide block 31 and the first spring 33 can bear a dynamic load with higher strength, and has higher reliability. In a preferred embodiment, a pair of relatively parallel actuating mechanisms 11 is shown, but it should be understood that it is also feasible to set only one actuating mechanism or more actuating mechanisms. The support plate 25 can be fixed on the slide block 31 by screws (not shown) which pass through the mounting holes 27 on the support plate 25 of the movable unit 9 and are screwed into threaded holes 35 on the slide block 31, so that the support plate 25 can move together with the slide block 31. In this way, the support plate 25 (that is, the movable unit 9) can be automatically and quickly ejected under the action of the first spring 33.

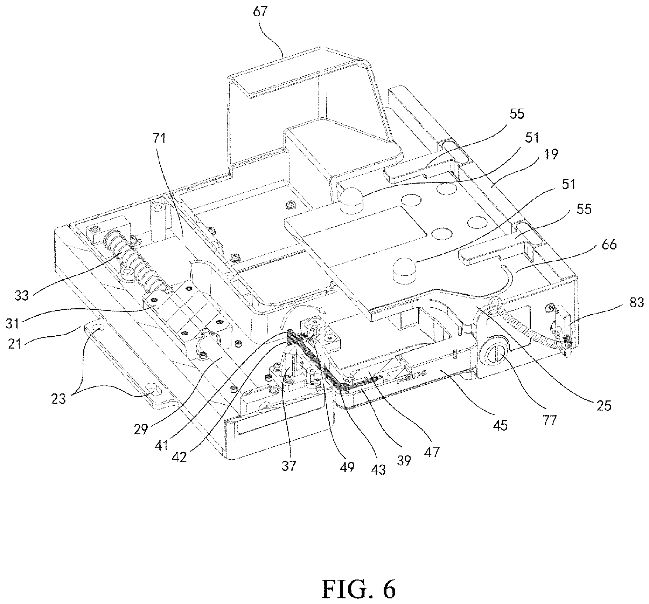

In particular, as shown in FIG. 5 and FIG. 6, the locking mechanism 13 for locking the movable unit 9 moved to the inside of the base 7 in place includes a locking block arranged on the base plate 17, a locking rod mounted on the movable unit 9 and capable of engaging with the locking block so as to lock the movable unit 9 in a working position, and an operating component for operating the locking rod to disengage the locking rod from the engagement with the locking block. In a preferred embodiment, two locking blocks 37 arranged on the base plate 17 and two L-shaped locking rods 39 mounted on the movable unit 9 are provided, each of the L-shaped locking rods 39 is provided with a hook 41 at a first end close to the corresponding locking block 37, and each of the L-shaped locking rods 39 is pivotally mounted on the movable unit 9 by a pivot 43 at a middle corner. In a preferred embodiment, each of the L-shaped locking rods 39 is mounted on a handle 45 for the support plate 25. The operating component includes a pressing component 47 mounted on the movable unit 9, especially on the handle 45, and capable of pressing a second end of each of the L-shaped locking rods 39. Each of the L-shaped locking rods 39 pivots around the pivot 43 under the load action of a second spring 49, so that the hook 41 formed at a first end of the locking rod is hooked on the corresponding locking block 37, and a second end of the locking rod abuts against the pressing component 47. In this way, the second end of the L-shaped locking rod 39 is pressed by the pressing component 47, causing the L-shaped locking rod 39 to pivot against the action of the second spring 49, so that the hook 41 at the first end of the L-shaped locking rod 39 disengages from the locking block 37 to allow the movable unit 9 to move together with the slide block 31. The L-shaped locking rod 39 is designed as a spring-loaded lever which converts a linear motion into a rotary motion so as to reduce the friction between the hook 41 and the locking block 37 in an unlocking process, so that an unlocking operation is smoother and more convenient. In order to facilitate the hook 41 to move to pass through the locking block 37 when the movable unit 9 is pushed back to the inside of the base 7, the hook 41 has an inclined surface 42 in contact with the locking block 37 to guide the hook 41 to move.

In order to facilitate the medical staff to grasp, a notch 48 can be formed on the support plate 25 so as to mount the handle 45 in the notch 48, and correspondingly, another notch 50 can be formed on the base plate 17. The pressing component 47 can be preferably arranged on the inside of the handle 45 to prevent accidental contact with the pressing component 47, causing the hook 41 at the first end of the L-shaped locking rod 39 to disengage from the locking block 37.

As shown in FIG. 1 to FIG. 4 and FIG. 6, the stop mechanism 15 for preventing the adapter plate 5 mounted on the movable unit 9 from moving includes convex pillars 51 arranged on the support plate 25, and the convex pillars 51 can be inserted into positioning holes 53 formed on the adapter plate 5, so as to limit the adapter plate 5 from horizontally moving relative to the support plate 25. In a preferred embodiment, four convex pillars 51 are arranged on the support plate 25, and four corresponding positioning holes 53 are arranged on the adapter plate 5. However, it should be understood that the number of the convex pillars 51 and the corresponding positioning holes 53 may be less than or more than four. A top end of each of the convex pillars 51 can be designed to be hemispherical to realize quick and accurate introduction of the positioning hole 53 on the adapter plate 5, thereby realizing quick mounting of the defibrillator. In addition, in a preferred embodiment, the convex pillar 51 is substantially cylindrical, and the corresponding positioning hole 53 is a circular hole. It should be understood that the convex pillar 51 may be of other shapes, such as a prismatic shape, and the positioning hole 53 may be a square hole. Moreover, it is also feasible to arrange the convex pillars on the adapter plate and form the positioning holes on the support plate. The stop mechanism 15 for preventing the adapter plate 5 mounted on the movable unit 9 from moving further includes stop rods 55 arranged on the two side plates 19 of the base 7 respectively and extending toward the inside of the base 7. The stop rod 55 has a certain distance from the support plate 25 in a vertical direction to allow the movable unit 9 and the adapter plate 5 mounted on the movable unit 9 to horizontally move, and limit the adapter plate 5 from vertically moving relative to the support plate 25 after the adapter plate 5 horizontally moves under the stop rod 55 (that is, the movable unit 9 and the adapter plate 5 are moved to the inside of the base 7). Therefore, two stop rods 55 can be arranged at an interval on each of the side plates 19, the adapter plate 5 is preferably designed to be I-shaped, and each arm of the I-shaped adapter plate 5 is provided with a stepped recess 57. When the movable unit 9 with the adapter plate 5 is inserted into the inside of the base 7, each of the stop rods 55 is respectively received in the corresponding stepped recess 57, so as to prevent the adapter plate 5 from vertically moving relative to the support plate 25.

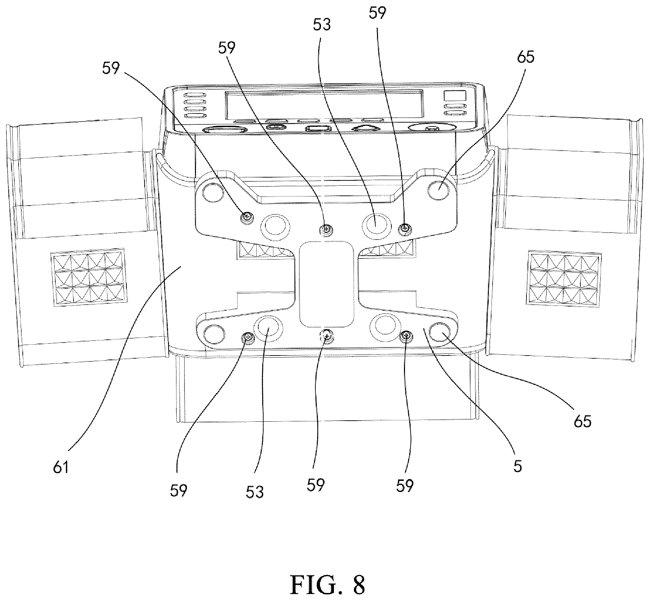

FIG. 8 schematically shows an adapter plate of a device for fixing a defibrillator according to the present invention, mounted on a bottom of the defibrillator, by a three-dimensional view. As shown in FIG. 8, the I-shaped adapter plate 5 is fixed to the bottom of a defibrillator 61 by a plurality of screws 59, so as to connect the I-shaped adapter plate 5 with the defibrillator 61 as a whole. In order to realize uniform stress of the I-shaped adapter plate 5 and the defibrillator 61 when the I-shaped adapter plate 5 is connected to the defibrillator 61, as shown in FIG. 1, a plurality of buffer pads 63 can be arranged on a surface where the I-shaped adapter plate 5 is in contact with the defibrillator 61. Similarly, as shown in FIG. 8, a plurality of additional buffer pads 65 can be arranged on a surface where the I-shaped adapter plate 5 is in contact with the support plate 25. When the I-shaped adapter plate 5 and the defibrillator which are connected as a whole are mounted on the support plate 25, the defibrillator may affect the quick alignment between the convex pillar 51 on the support plate 25 and the positioning hole 53 on the adapter plate 5. As a result, a guide groove 66 corresponding to the outer contour of the adapter plate 5 can be formed on the support plate 25 to facilitate the quick alignment between the convex pillar 51 and the positioning hole 53.

As shown in FIG. 1 and FIG. 3, a wire guard 67 can be arranged at a rear end of the base 7, and the wire guard 67 is provided with a wire hole for a wire for supplying power to the defibrillator 61 to pass through. The wire guard 67 can be configured to accommodate the wire, prevent the medical staff from accidentally touching a power supply and causing an electric shock when the defibrillator 61 runs, and also prevent the defibrillator from being unstable in a vibration environment of an ambulance.

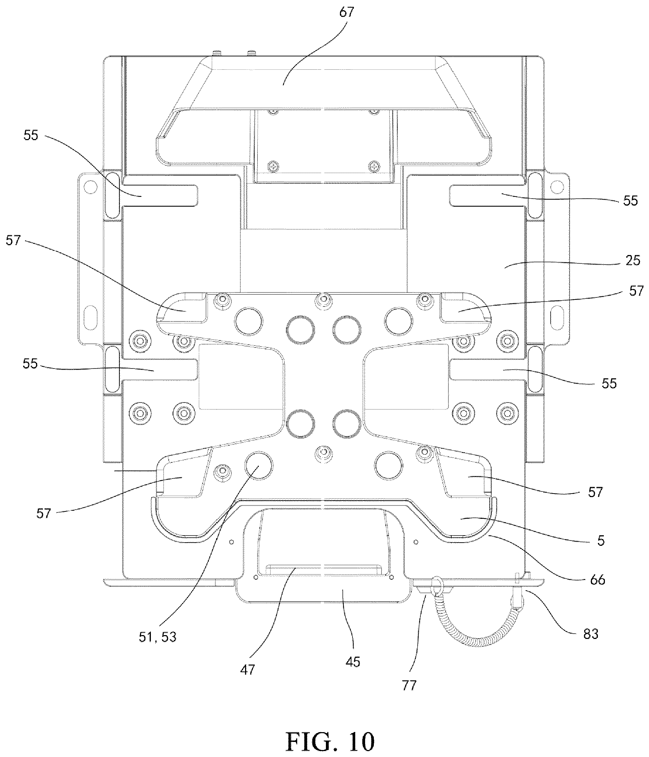

The base 7 can further be provided with a dustproof wall 71, and the dustproof wall 71, the base 7, and the support plate 25 together define a substantially closed space for accommodating the actuating mechanism 11. As a result, as shown in FIG. 10, the components of the actuating mechanism 11 are not exposed to an external environment. In this way, external dust can be prevented from contaminating the components of the actuating mechanism, and moreover, blood or body fluid can be prevented from being splashed onto the surfaces of the components of the actuating mechanism and cannot be cleaned. The dustproof wall 71 and the base 7 can be integrally formed, and preferably integrally cast into an aluminum frame.

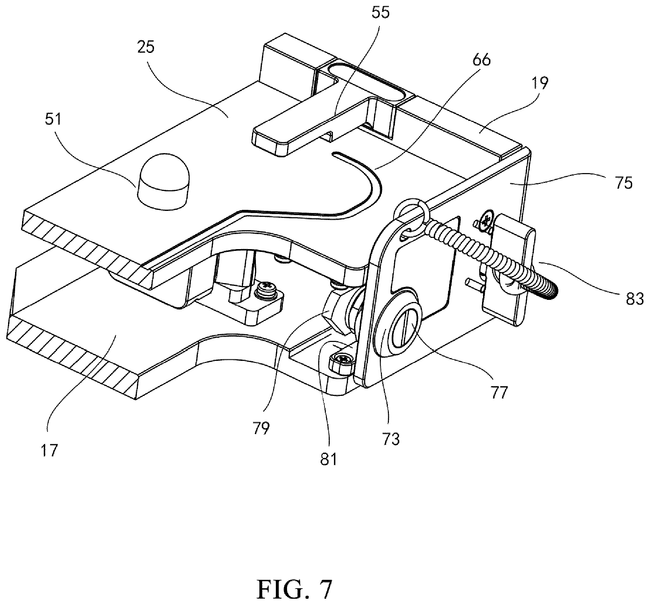

Although the locking mechanism 13 is provided, in order to prevent the fixing failure of the adapter plate and the defibrillator mounted on the movable unit 9 due to the misoperation of the pressing component on the handle, an independent lock 73 can be additionally provided. FIG. 7 schematically shows an independent lock of a device for fixing a defibrillator according to the present invention by a partial three-dimensional view. As shown in FIG. 7, the independent lock 73 includes a lock body 77 mounted on a front-end plate 75 of the movable unit 9, a bolt 79 mounted on the lock body 77, and a stop block 81 mounted on the base plate 17 of the base 7. The front-end plate 75 of the movable unit 9 is mounted at a front-end of the support plate 25 of the movable unit 9. The independent lock 73 further includes a key 83. When not in use, the key 83 can be inserted into a key receiving part 85 on the front-end plate 75. When in use, the key 83 is pulled out from the key receiving part 85 and inserted into the lock body 77 to rotate the lock body 77 and the bolt 79 mounted thereon. When the bolt 79 is rotated to the rear of the stop block 81 on the base plate 17, the movable unit 9 cannot be ejected even if the pressing component is operated. Only after the bolt 79 is rotated to move away from the rear of the stop block 81 on the base plate 17, the movable unit 9 can be ejected by operating the pressing component, thereby preventing any possible misoperation.

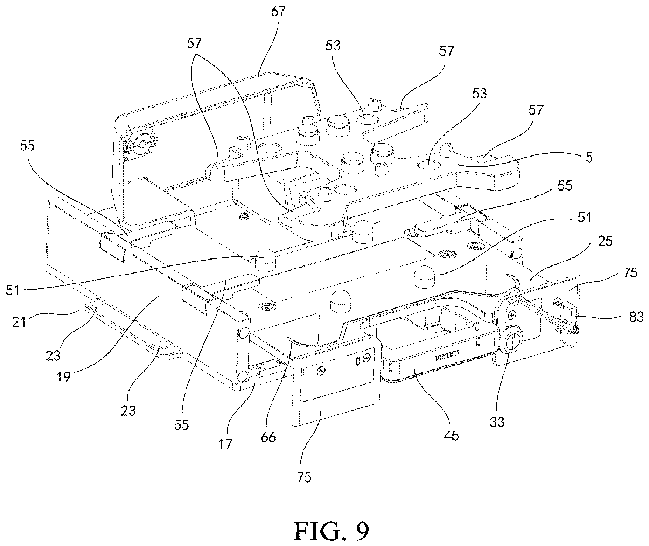

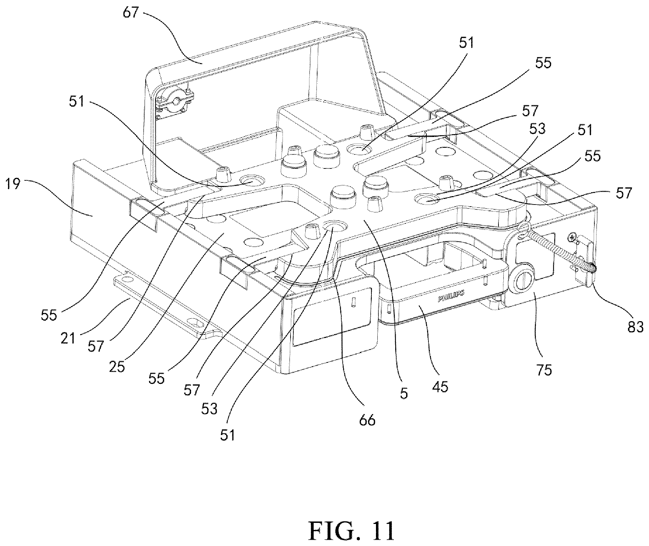

FIG. 9 schematically shows a device for fixing a defibrillator according to the present invention by a three-dimensional view, wherein a movable unit is ejected from a base and an adapter plate is not placed on the movable unit. FIG. 10 is a top view of the device for fixing a defibrillator in a state of FIG. 9. FIG. 11 schematically shows a device for fixing a defibrillator according to the present invention by a three-dimensional view, wherein a movable unit is inserted into a base and an adapter plate is fixedly held on the movable unit. The operation of the device for fixing a defibrillator according to the present invention will be described below with reference to FIG. 9 to FIG. 11. In order to clearly show the operation of the device for fixing a defibrillator according to the present invention, the defibrillator 61 mounted on the adapter plate 5 is removed in FIG. 9 to FIG. 11.

Under normal conditions, the device for fixing a defibrillator according to the present invention is fixedly mounted on an ambulance by screws (not shown) passing through the through holes 23 on the side wings 21 protruding from both sides of the base plate 17. When the defibrillator needs to be used, the medical staff first pulls out the key 83 and inserts it into the lock body 77 to rotate the lock body 77 and the bolt 79 mounted thereon, so that the bolt 79 moves away from the rear of the stop block 81 on the base plate 17. Then, the medical staff holds the handle 45 to compress the pressing component 47 to cause the L-shaped locking rod 39 to pivot against the action of the second spring 49, so that the hook 41 at the first end of the L-shaped locking rod 39 is disengaged from the locking block 37. As a result, under the action of the first spring 33 abutting against the slide block 31, the movable unit 9 including the support plate 25 and the front-end plate 75 is automatically and quickly ejected from the base 7 to the outside of the base 7. At this time, as shown in FIG. 9 and FIG. 10, the support plate 25 is moved to a position where the stop rod 55 on the side plate 19 of the base 7 does not prevent the convex pillar 51 on the support plate 25 from being inserted into the positioning hole 53 on the I-shaped adapter plate 5. Then, according to the prompt of the guide groove 66, the convex pillar 51 on the support plate 25 is quickly aligned with the positioning hole 53 on the adapter plate 5 and inserted therein, so as to mount the I-shaped adapter plate 5 and the defibrillator which are connected as a whole on the support plate 25. Subsequently, against the action of the first spring 33, the movable unit 9 provided with the I-shaped adapter plate 5 and the defibrillator is pushed back to the inside of the base 7 until the hook 41 of the L-shaped locking rod 39 of the locking mechanism 13 is hooked on the corresponding locking block 37 under the action of the second spring 49. At this time, the movable unit 9 provided with the I-shaped adapter plate 5 and the defibrillator is moved to a position as shown in FIG. 11 where each of the stop rods 55 on the side plate 19 of the base 7 is received in the corresponding stepped recess 57 on each arm of the I-shaped adapter plate 5, so as to prevent the adapter plate 5 from vertically moving relative to the support plate 25. Finally, the bolt 79 on the lock body 77 is rotated to the rear of the stop block 81 on the base plate 17 by the key 83. In this way, the defibrillator can be conveniently, quickly, firmly, and reliably fixed on the ambulance.

Although the present invention is described in detail with reference to the preferred embodiments of the present invention, it should be understood that the detailed description is only used for explaining the present invention, but does not constitute a limitation of the present invention. The scope of the present invention is determined by the technical solution defined in claims.

## Claims

1. A device for fixing a defibrillator, comprising:
a base assembly (3); and
an adapter plate (5) detachably mounted on the base assembly (3), the adapter plate (5) being fixedly mounted on the defibrillator;
wherein the base assembly (3) comprises:
a base (7) comprising a base plate (17);
a movable unit (9), the adapter plate (5) being detachably mounted on the movable unit (9);
an actuating mechanism (11) mounted on the base (7) and configured to drive the movable unit (9) toward the outside of the base (7);
a locking mechanism (13) for locking the movable unit (9) moved to the inside of the base (7) in place; and
a stop mechanism (15) for preventing the adapter plate (5) mounted on the movable unit (9) from moving,
**characterized in that**
the movable unit (9) comprises a support plate (25) for mounting the adapter plate (5);
the actuating mechanism (11) comprises a guide rod (29) fixedly supported on the base plate (17), a slide block (31) slidably arranged on the guide rod (29), and a first spring (33) sheathed on the guide rod (29) and exerting a spring force on the slide block (31) so as to push the slide block (31) along the guide rod (29); and
the support plate (25) is fixed on the slide block (31) so as to move together with the slide block (31).

2. The device for fixing a defibrillator according to claim 1, wherein the locking mechanism (13) comprises a locking block arranged on the base plate (17), a locking rod mounted on the movable unit (9) and capable of engaging with the locking block, and an operating component for operating the locking rod to disengage the locking rod from the engagement with the locking block.

3. The device for fixing a defibrillator according to claim 1, wherein the locking mechanism (13) comprises:
two locking blocks (37) arranged on the base plate (17);
two L-shaped locking rods (39) mounted on the movable unit (9), each of the L-shaped locking rods (39) being provided with a hook (41) at a first end close to the corresponding locking block (37), and each of the L-shaped locking rods (39) being pivotally mounted on the movable unit (9) at a corner;
a pressing component (47) mounted on the movable unit (9) and capable of pressing a second end of each of the L-shaped locking rods (39); and
second springs (49) acting on each of the L-shaped locking rods (39) respectively, the second springs (49) enabling each of the L-shaped locking rods (39) to pivot so that the hook (41) is hooked on the corresponding locking block (37).

4. The device for fixing a defibrillator according to claim 3, wherein the support plate (25) is provided with a handle (45), and each of the L-shaped locking rods (39) and the pressing component (47) are mounted on the handle (45).

5. The device for fixing a defibrillator according to claim 4, wherein the pressing component (47) is arranged on the inside of the handle (45).

6. The device for fixing a defibrillator according to claim 1, wherein the base (7) further comprises side plates (19) fixedly arranged at both sides of the base plate (17), and the stop mechanism (15) comprises:
convex pillars (51) arranged on the support plate (25), the convex pillars (51) being inserted into positioning holes (53) formed on the adapter plate (5) to limit the adapter plate (5) from horizontally moving relative to the support plate (25); and
stop rods (55) arranged on each of the side plates (19) respectively and extending toward the inside of the base (7), the stop rod (55) having a certain distance from the support plate (25) in a vertical direction to allow the movable unit (9) and the adapter plate (5) mounted on the movable unit (9) to horizontally move, and limit the adapter plate (5) from vertically moving relative to the support plate (25) when the movable unit (9) and the adapter plate (5) move to the inside of the base (7).

7. The device for fixing a defibrillator according to claim 6, wherein a top end of the convex pillar (51) is designed to be hemispherical.

8. The device for fixing a defibrillator according to claim 6, wherein the adapter plate (5) is designed to be I-shaped, each arm of the adapter plate (5) is provided with a stepped recess (57), and when the movable unit (9) and the adapter plate (5) are moved to the inside of the base (7), the stop rod (55) is respectively received in the corresponding stepped recess (57).

9. The device for fixing a defibrillator according to claim 6, wherein the support plate (25) is provided with a guide groove (66) corresponding to the outer contour of the adapter plate (5).

10. The device for fixing a defibrillator according to claim 1, wherein a wire guard (67) is arranged at a rear end of the base (7), and a wire hole is formed on the wire guard (67).

11. The device for fixing a defibrillator according to claim 1, wherein the base (7) is provided with a dustproof wall (71), and the dustproof wall (71), the base (7), and the support plate (25) together define a closed space for accommodating the actuating mechanism (11).

12. The device for fixing a defibrillator according to claim 1, **characterized by** further comprising an independent lock (73) for preventing the movable unit (9) from being accidentally ejected, wherein the movable unit (9) further comprises a front-end plate (75) arranged at a front-end of the support plate (25), and the independent lock (73) comprises a bolt (79) arranged on the front-end plate (75) and a stop block (81) arranged on the base plate (17) and configured to block the bolt (79).

## Patentansprüche

1. Vorrichtung zur Befestigung eines Defibrillators, umfassend:
eine Basisanordnung (3); und
eine Adapterplatte (5), die abnehmbar an der Basisanordnung (3) montiert ist, wobei die Adapterplatte (5) fest am Defibrillator montiert ist;
wobei die Basisanordnung (3) Folgendes umfasst:
eine Basis (7), die eine Basisplatte (17) umfasst;
eine bewegliche Einheit (9), wobei die Adapterplatte (5) abnehmbar an der beweglichen Einheit (9) montiert ist;
einen Betätigungsmechanismus (11), der auf der Basis (7) montiert ist und dazu konfiguriert ist, die bewegliche Einheit (9) in Richtung der Außenseite der Basis (7) anzutreiben;
einen Verriegelungsmechanismus (13) zum Verriegeln der beweglichen Einheit (9), die zu der Innenseite der Basis (7) bewegt wurde, an Ort und Stelle; und
einen Anschlagmechanismus (15) zum Verhindern einer Bewegung der auf der beweglichen Einheit (9) montierten Adapterplatte (5),
**dadurch gekennzeichnet, dass**
die bewegliche Einheit (9) eine Trägerplatte (25) zum Montieren der Adapterplatte (5) umfasst;
der Betätigungsmechanismus (11) eine Führungsstange (29) umfasst, die fest auf der Basisplatte (17) getragen wird, einen Gleitblock (31), der gleitbar auf der Führungsstange (29) angeordnet ist, und eine erste Feder (33), die auf der Führungsstange (29) ummantelt und eine Federkraft auf den Gleitblock (31) ausübt, um den Gleitblock (31) entlang der Führungsstange (29) zu schieben; und
die Trägerplatte (25) auf dem Gleitblock (31) befestigt ist, um sich gemeinsam mit dem Gleitblock (31) zu bewegen.

2. Vorrichtung zur Befestigung eines Defibrillators nach Anspruch 1, wobei der Verriegelungsmechanismus (13) einen auf der Basisplatte (17) angeordneten Verriegelungsblock, eine an der beweglichen Einheit (9) montierte und mit dem Verriegelungsblock in Eingriff bringbare Verriegelungsstange und eine Betriebskomponente zum Betreiben der Verriegelungsstange umfasst, um die Verriegelungsstange aus dem Eingriff mit dem Verriegelungsblock zu lösen.

3. Vorrichtung zur Befestigung eines Defibrillators nach Anspruch 1, wobei der Verriegelungsmechanismus (13) Folgendes umfasst:
zwei auf der Basisplatte (17) angeordnete Verriegelungsblöcke (37);
zwei L-förmige Verriegelungsstangen (39), die an der beweglichen Einheit (9) montiert sind, wobei jede der L-förmigen Verriegelungsstangen (39) an einem ersten Ende in der Nähe des entsprechenden Verriegelungsblocks (37) mit einem Haken (41) bereitgestellt ist und jede der L-förmigen Verriegelungsstangen (39) an einer Ecke schwenkbar an der beweglichen Einheit (9) montiert ist;
eine Andrückkomponente (47), das an der beweglichen Einheit (9) montiert ist und auf ein zweites Ende jeder der L-förmigen Verriegelungsstangen (39) drücken kann; und
zweite Federn (49), die jeweils auf jede der L-förmigen Verriegelungsstangen (39) wirken, wobei die zweiten Federn (49) es ermöglichen, dass jede der L-förmigen Verriegelungsstangen (39) so schwenkt, dass der Haken (41) an dem entsprechenden Verriegelungsblock (37) eingehakt wird.

4. Vorrichtung zur Befestigung eines Defibrillators nach Anspruch 3, wobei die Trägerplatte (25) mit einem Griff (45) bereitgestellt ist und jede der L-förmigen Verriegelungsstangen (39) und die Andrückkomponente (47) am Griff (45) montiert sind.

5. Vorrichtung zur Befestigung eines Defibrillators nach Anspruch 4, wobei die Andrückkomponente (47) an der Innenseite des Griffs (45) angeordnet ist.

6. Vorrichtung zur Befestigung eines Defibrillators nach Anspruch 1, wobei die Basis (7) weiter Seitenplatten (19) umfasst, die fest an beiden Seiten der Basisplatte (17) angeordnet sind, und wobei der Anschlagmechanismus (15) Folgendes umfasst:
konvexe Säulen (51), die auf der Trägerplatte (25) angeordnet sind, wobei die konvexen Säulen (51) in Positionierungslöcher (53) eingesetzt sind, die auf der Adapterplatte (5) gebildet sind, um eine horizontale Bewegung der Adapterplatte (5) zur Trägerplatte (25) relativ zu begrenzen; und
Anschlagstangen (55), die jeweils an jeder der Seitenplatten (19) angeordnet sind und sich in Richtung der Innenseite der Basis (7) erstrecken, wobei die Anschlagstange (55) in vertikaler Richtung einen bestimmten Abstand von der Trägerplatte (25) aufweist, um eine horizontale Bewegung der beweglichen Einheit (9) und der auf der beweglichen Einheit (9) montierten Adapterplatte (5) zu ermöglichen und eine vertikale Bewegung der Adapterplatte (5) zur Trägerplatte (25) relativ zu begrenzen, wenn sich die bewegliche Einheit (9) und die Adapterplatte (5) in die Innenseite der Basis (7) bewegen.

7. Vorrichtung zur Befestigung eines Defibrillators nach Anspruch 6, wobei ein oberes Ende der konvexen Säule (51) halbkugelförmig ausgebildet ist.

8. Vorrichtung zur Befestigung eines Defibrillators nach Anspruch 6, wobei die Adapterplatte (5) I-förmig ausgebildet ist, jeder Schenkel der Adapterplatte (5) mit einer stufenförmigen Aussparung (57) bereitgestellt ist und beim Bewegen der beweglichen Einheit (9) und der Adapterplatte (5) in das Innere der Basis (7) die Anschlagstange (55) jeweils in der entsprechenden stufenförmigen Aussparung (57) aufgenommen wird.

9. Vorrichtung zur Befestigung eines Defibrillators nach Anspruch 6, wobei die Trägerplatte (25) mit einer der Außenkontur der Adapterplatte (5) entsprechenden Führungsnut (66) bereitgestellt ist.

10. Vorrichtung zur Befestigung eines Defibrillators nach Anspruch 1, wobei an einem hinteren Ende der Basis (7) ein Drahtschutz (67) angeordnet ist und an dem Drahtschutz (67) ein Drahtloch gebildet ist.

11. Vorrichtung zur Befestigung eines Defibrillators nach Anspruch 1, wobei die Basis (7) mit einer staubdichten Wand (71) bereitgestellt ist, und die staubdichte Wand (71), die Basis (7) und die Trägerplatte (25) zusammen einen geschlossenen Raum zur Aufnahme des Betätigungsmechanismus (11) definieren.

12. Vorrichtung zur Befestigung eines Defibrillators nach Anspruch 1, **dadurch gekennzeichnet, dass** sie weiter eine unabhängige Verriegelung (73) umfasst, um ein versehentliches Herausschleudern der beweglichen Einheit (9) zu verhindern, wobei die bewegliche Einheit (9) weiter eine vordere Endplatte (75) umfasst, die an einem vorderen Ende der Trägerplatte (25) angeordnet ist, und die unabhängige Verriegelung (73) einen Bolzen (79) umfasst, der an der vorderen Endplatte (75) angeordnet ist, sowie einen Anschlagblock (81), der an der Basisplatte (17) angeordnet ist und zum Blockieren des Bolzens (79) konfiguriert ist.

## Revendications

1. Dispositif de fixation d'un défibrillateur, comprenant :
un ensemble formant base (3) ;
une plaque adaptatrice (5) montée de manière amovible sur l'ensemble formant base (3), la plaque adaptatrice (5) étant montée de manière fixe sur le défibrillateur ;
dans lequel l'ensemble formant base (3) comprend :
une base (7) comprenant une plaque de base (17) ;
une unité mobile (9), la plaque adaptatrice (5) étant montée de manière amovible sur l'unité mobile (9) ;
un mécanisme d'actionnement (11) monté sur la base (7) et configuré pour entraîner l'unité mobile (9) vers l'extérieur de la base (7) ;
un mécanisme de verrouillage (13) pour verrouiller l'unité mobile (9) déplacée vers l'intérieur de la base (7) en place ; et
un mécanisme d'arrêt (15) pour empêcher la plaque adaptatrice (5) montée sur l'unité mobile (9) de se déplacer,
**caractérisé en ce que**
l'unité mobile (9) comprend une plaque de support (25) pour le montage de la plaque adaptatrice (5) ;
le mécanisme d'actionnement (11) comprend une tige de guidage (29) supportée fixement sur la plaque de base (17), un bloc coulissant (31) disposé de manière coulissante sur la tige de guidage (29) et un premier ressort (33) gainé sur la tige de guidage (29) et exerçant une force de ressort sur le bloc coulissant (31) de manière à pousser le bloc coulissant (31) le long de la tige de guidage (29) ; et
la plaque de support (25) est fixée sur le bloc coulissant (31) de manière à se déplacer conjointement avec le bloc coulissant (31).

2. Dispositif de fixation d'un défibrillateur selon la revendication 1, dans lequel le mécanisme de verrouillage (13) comprend un bloc de verrouillage disposé sur la plaque de base (17), une tige de verrouillage montée sur l'unité mobile (9) et capable de se mettre en prise avec le bloc de verrouillage et un composant de fonctionnement pour faire fonctionner la tige de verrouillage afin de désengager la tige de verrouillage de la mise en prise avec le bloc de verrouillage.

3. Dispositif de fixation d'un défibrillateur selon la revendication 1, dans lequel le mécanisme de verrouillage (13) comprend :
deux blocs de verrouillage (37) disposés sur la plaque de base (17) ;
deux tiges de verrouillage en forme de L(39) montées sur l'unité mobile (9), chacune des tiges de verrouillage en forme de L (39) étant munie d'un crochet (41) au niveau d'une première extrémité proche du bloc de verrouillage correspondant (37) et chacune des tiges de verrouillage en forme de L (39) étant montée pivotante sur l'unité mobile (9) au niveau d'un coin ;
un composant de pression (47) monté sur l'unité mobile (9) et capable de presser une seconde extrémité de chacune des tiges de verrouillage en L (39) ; et
des seconds ressorts (49) agissant respectivement sur chacune des tiges de verrouillage en L (39), les seconds ressorts (49) permettant à chacune des tiges de verrouillage en L (39) de pivoter de sorte que le crochet (41) est accroché sur le bloc de verrouillage correspondant (37).

4. Dispositif de fixation d'un défibrillateur selon la revendication 3, dans lequel la plaque de support (25) est munie d'une poignée (45) et chacune des tiges de verrouillage en forme de L (39) et le composant de pression (47) sont montés sur la poignée (45).

5. Dispositif de fixation d'un défibrillateur selon la revendication 4, dans lequel l'élément de pression (47) est disposé à l'intérieur de la poignée (45).

6. Dispositif de fixation d'un défibrillateur selon la revendication 1, dans lequel la base (7) comprend en outre des plaques latérales (19) disposées de manière fixe de part et d'autre de la plaque de base (17) et le mécanisme d'arrêt (15) comprend :
des piliers convexes (51) disposés sur la plaque de support (25), les piliers convexes (51) étant insérés dans des trous de positionnement (53) formés sur la plaque adaptatrice (5) pour limiter le mouvement horizontal de la plaque adaptatrice (5) par rapport à la plaque de support (25) ; et
des tiges d'arrêt (55) disposées sur chacune des plaques latérales (19) respectivement et s'étendant vers l'intérieur de la base (7), la tige d'arrêt (55) présentant une certaine distance de la plaque de support (25) dans une direction verticale pour permettre à l'unité mobile (9) et à la plaque adaptatrice (5) montée sur l'unité mobile (9) de se déplacer horizontalement et limiter le déplacement vertical de la plaque adaptatrice (5) par rapport à la plaque de support (25) lorsque l'unité mobile (9) et la plaque adaptatrice (5) se déplacent vers l'intérieur de la base (7).

7. Dispositif de fixation d'un défibrillateur selon la revendication 6, dans lequel une extrémité supérieure du pilier convexe (51) est conçue pour être hémisphérique.

8. Dispositif de fixation d'un défibrillateur selon la revendication 6, dans lequel la plaque adaptatrice (5) est conçue en forme de I, chaque bras de la plaque adaptatrice (5) est pourvu d'un évidement étagé (57) et lorsque l'unité mobile (9) et la plaque adaptatrice (5) sont déplacées vers l'intérieur de la base (7), la tige de butée (55) est respectivement reçue dans l'évidement étagé correspondant (57).

9. Dispositif de fixation d'un défibrillateur selon la revendication 6, dans lequel la plaque de support (25) est pourvue d'une rainure de guidage (66) correspondant au contour extérieur de la plaque adaptatrice (5).

10. Dispositif de fixation d'un défibrillateur selon la revendication 1, dans lequel un protège-fil (67) est disposé au niveau d'une extrémité arrière de la base (7) et un trou de fil est formé sur le protège-fil (67).

11. Dispositif de fixation d'un défibrillateur selon la revendication 1, dans lequel la base (7) est pourvue d'une paroi anti-poussière (71) et la paroi anti-poussière (71), la base (7) et la plaque de support (25) définissent conjointement un espace fermé pour loger le mécanisme d'actionnement (11).

12. Dispositif de fixation d'un défibrillateur selon la revendication 1, **caractérisé en ce qu'**il comprend en outre un verrou indépendant (73) pour empêcher l'éjection accidentelle de l'unité mobile (9), dans lequel l'unité mobile (9) comprend en outre une plaque d'extrémité avant (75) disposée au niveau d'une extrémité avant de la plaque de support (25) et le verrou indépendant (73) comprend un boulon (79) disposé sur la plaque d'extrémité avant (75) et un bloc d'arrêt (81) disposé sur la plaque de base (17) et configuré pour bloquer le boulon (79).
